# EUROPEAN PATENT SPECIFICATION

(11) **EP 1 834 663 B1**
(45) Date of publication and mention of the grant of the patent: **01.10.2008**
(21) Application number: 07002923.6
(22) Date of filing: 12.02.2007
(51) Int. Cl.: A61M 25/01

(54) **Medical tube**
Medizinischer Schlauch
Tube médical

(30) Priority: 17.03.2006 JP 2006075184
(43) Date of publication of application: 19.09.2007
(73) Proprietor: Covidien AG, 8212 Neuhausen am Rheinfall (CH)
(72) Inventor: Abe, Kazuhiro, Fukuroi-shi, Shizuoka-ken (JP); Hayakawa, Toshinobu, Fukuroi-shi, Shizuoka-ken (JP); Sakai, Yosuke, Fukuroi-shi, Shizuoka-ken (JP); Hoshinouchi, Yuya, Fukuroi-shi, Shizuoka-ken (JP)
(74) Representative: Tomlinson, Edward James

(56) References cited:
- JP-A- 3 118 760
- US-A- 3 788 304
- US-A- 5 083 549
- US-A- 5 938 588
- US-A- 6 066 090

## Description

The present invention pertains to a medical tube for insertion into the body, especially body cavities that are used for feeding a medicinal solution, etc., to the body or discharging bodily fluids such as waste, etc.

### Prior art

Various kinds of tubes are used medically inside the body. Among them, ileus tubes are used for diagnosis or treatment of intestinal obstruction. An ileus tube is inserted nasally or orally to the proper site such as the small intestine, etc., and used for pressure reduction in the affected site, suction, injection of medicinal solutions such as contrast agents or washing fluids, etc. This ileus tube has to pass through the pyloric ring and Treitz's ligament before reaching the affected site such as the small intestine, etc. These regions bend and/or narrow, and it is difficult to pass an ileus tube through them. Therefore, various medical tubes with various devices used to pass through these regions have been proposed.

A medical tube according to the preamble of claim 1 is described for example in US-A-5 083 549.

Japanese Kokai Utility Model No. Hei 3[1991]-118760 discloses a medical tube with a weight at the tip. The tube tip is bendable because of the weight attached, allowing the tube to pass through curved body cavities smoothly. Furthermore, Japanese Kokai Patent Application No. Hei 5[1993]-345031 discloses a medical tube made of a hard resin material except for the tip, which is made of a soft resin material, where the tip is actively bendable by pulling a maneuvering wire installed along the tube wall.

### Disclosure of the invention

The medical tube disclosed in Patent Reference 1 may pass through curved cavities inside the body by utilizing the weighed tip portion, which is bendable due to the force of gravity. Consequently, each time the bending direction of the path changes, adjustment is necessary to match the bending direction of the path, typically by changing the position of the patient, which stresses the patient. On the other hand, in the medical tube disclosed in Japanese Kokai Patent Application No. Hei 5[1993]-345031, the tip may be actively turned in the desired direction by pulling the maneuvering wire, allowing the medical tube to pass any curving path inside the body while maintaining the posture of the patient. However, this medical tube is prepared by joining a tip made of a soft resin material and main body made of a hard resin material, and consequently, the production cost is high.

The present invention was developed under the circumstances described above. The object is to provide a medical tube that can be produced at a low cost and that can be passed through the path of curved cavities inside the body.

To accomplish the above object, the medical tube of the present invention equipped with a main body tube portion with a main interior lumen and guide tube portion, which has an outer tube diameter smaller than that of the main body tube, is of the same material as that forming the main body tube portion, and is formed as a single body with the main body tube portion at one end; having maneuvering wire lumens formed at radial positions deviating from the central axis of the guide and main tube portions from the guide tube portion to the main body tube portion along the axial direction; and the maneuvering wire lumens having inserted maneuvering wires, the ends of which are attached to the guide tube portion. One or more weights are attached to the guide tube portion around its periphery.

The medical tube of the present invention as described above has a main body tube portion and guide tube portion connected to one end of the main body tube. Furthermore, the outer diameter of the guide tube is smaller than that of the main body tube. Consequently, the rigidity of the guide tube portion is lower than that of the main body tube portion, and it is easily bendable. Furthermore, maneuvering wire lumens are formed from the guide tube portion to the main body tube portion along the axial direction, and these maneuvering wire lumens are formed at radial positions deviating from the central axis of the guide tube and main body tube portions. Therefore, when the maneuvering wires inserted through the maneuvering wire lumens are tensioned, the tension is applied to the guide where the ends of the maneuvering wires are attached, deviating from the central axis enabling active bending of the guide tube. Consequently, it becomes possible to allow the medical tube inserted in a cavity inside the body to advance through a curved path by applying tension to the maneuvering wires and bending the guide tube portion, depending on the extent of curvature of the path. In this case, the guide tube portion and main body tube portion are made of the same material as a unit; thus, processes of preparing the guide and main body tubes separately and joining them are not required. Thus, low-cost production is possible.

In this case, the medical tube of the present invention has multiple maneuvering wire lumens formed from the guide tube portion to the main body tube portion, and at least two or more of the maneuvering wire lumens among those multiple maneuvering wire lumens have inserted maneuvering wires. In this configuration, the multiple maneuvering wires are tensioned to allow bending of the guide tube portion in various directions. The suitable number of maneuvering wire lumens with inserted wires is in the range of 2-4; they are formed in the peripheral direction of the guide and main tube portions with an interval angle of 80-180° and equal spacing.

Furthermore, the medical tube of the present invention is desirably equipped with a tensioning means connected to the other ends of the maneuvering wires inserted in the multiple maneuvering wire lumens, to allow tensioning of the maneuvering wires. This tensioning means is used to pull a maneuvering wire, one end of which is connected to the guide tube portion; as a result, the maneuvering wire is tensioned, and it is possible to bend the guide tube portion in the desired direction. Furthermore, it is convenient because one tensioning means can adjust the tension of multiple maneuvering wires.

The tensioning means may be composed of multiple operation means, to which the other ends of the maneuvering wires inserted in the multiple maneuvering wire lumens are individually connected. By maneuvering the multiple operation means individually, the maneuvering wires corresponding to the operation means are tensioned, bending the guide tube portion in the desired direction. Furthermore, by simultaneously operating the multiple operation means, the multiple maneuvering wires are tensioned simultaneously to adjust the bending direction of the guide tube portion, and as a result, the guide tube portion may be bent in various directions.

Furthermore, the tensioning means may be connected to the other ends of all of the maneuvering wires inserted in the maneuvering wire lumens and having an operation means, thus tensioning a specific maneuvering wire depending on the state of operation. For example, the operation means may be a maneuvering lever such as a joystick, and it is possible to configure tension adjustment for each maneuvering wire by pushing and bending this lever in any desired direction. As a result, all of the maneuvering wires are operable with one operation means, and the process of bending the guide tube portion can be carried out easily.

### Brief description of the figures

Preferred embodiments of the invention will now be described, by way of examole only, with reference to the accompanying drawings in which:
Figure 1 is a plan view of an ileus tube in the embodiment of the present invention;
Figure 2 is a cross section of Figure 1 along line A-A;
Figure 3 is an axial cross section of the main body tube and guide portion of an ileus tube in the embodiment of the present invention;
Figure 4 is an enlarged cross section of portion B of Figure 3;
Figure 5 is an enlarged cross section of portion C of Figure 3;
Figure 6 is a cross section of a tensioning means in the embodiment of the present invention;
Figure 7 is a cross section of Figure 6 along line D-D;
Figure 8 shows the bent state of the guide portion of an ileus tube in the embodiment of the present invention;
Figure 9 shows the state of the balloon of an ileus tube being expanded in the embodiment of the present invention;
Figure 10 is a plan view of an ileus tube having a different tensioning means;
Figure 11(a) is a front view including a partial cross section of a tensioning means in a different example. (b) is a front view of a tensioning means in a different example;
Figure 12 is a perspective view of a ball used in a tensioning means in a different example; and
Figure 13(a) is a front view including a partial cross section of a tensioning means in a different example. (b) is a front view of a tensioning means in a different example.

### Preferred embodiment of the invention

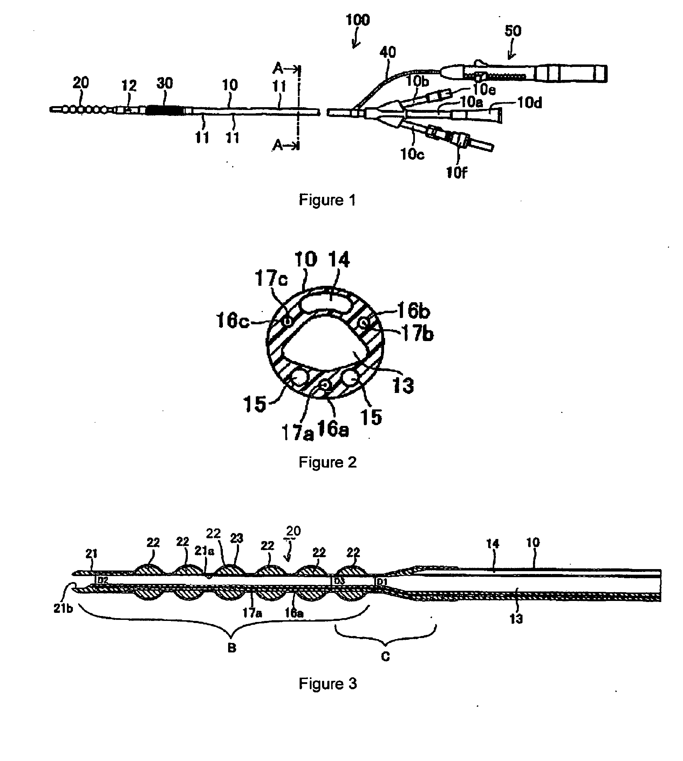

Figure 1 is a plan view showing the whole ileus tube as an example of the embodiment of the medical tube of the present invention. In the figure, an ileus tube 100 has a main body tube portion 10 and guide tube portion 20. The main body tube portion is, for example, 250-300 cm long with an outer diameter of 6 mm, and multiple lumens are formed inside in the axial direction. The base end of the main body tube portion 10 branches into 3 branch tubes (first branch 10a, second branch 10b and third branch 10c). Furthermore, one end of a connection tube 40 is connected to a site near the base of the main body tube portion 10, and the other end of this connection tube 40 is connected to a tensioning means 50.

The guide portion 20 of the ileus tube 100 is allowed to move forward inside a body cavity, for example when the ileus tube 100 is inserted nasally or orally, leading the main body tube portion 10 to the affected area, and it is installed on the tip side (left side in the figure) of the main body tube portion 10. A balloon 30 is attached to the periphery of the main body tube portion 10 on the left in the figure. This balloon 30 is expanded by supplying a fluid through a balloon lumen to be explained later, and in an area downstream from the stomach (such as the small intestine, duodenum, etc.), it comes into contact with the inner wall. Incidentally, more than one (e.g. 2) balloons 30 may be installed in ileus tube 100. Furthermore, the periphery of the main body tube portion 10 has multiple aspiration holes 11. In addition, the periphery of the main body tube portion 10 has, to the left of the balloon 30, an irrigation side hole 12.

Figure 2 is a cross section of Figure 1 along line A-A. It shows the radial cross section of the main body tube portion 10. As shown in Figure 2, a suction lumen 13, which is a lumen for discharging various materials from outside, is formed at about the center portion of the cross section of the main body tube portion 10. This suction lumen 13 is the main lumen of the present invention. Furthermore, formed below the suction lumen 13 in the figure are balloon lumens 15 feeding a fluid to balloon 30. In addition, on top of the suction lumen 13 in the figure, an irrigation lumen 14 is formed to feed air to the body cavity to prevent negative pressure in the body cavity. This irrigation lumen 14 may also be used to feed a medical solution such as a contrast agent, etc., in the body. Suction lumen 13 is formed along the axial direction of main body tube portion 10. Said aspiration holes 11 are connected to suction lumen 13. Two of the balloon lumens 15 are formed in parallel to suction lumen 13 along the axial direction of main body tube portion 10, and they are connected to balloon 30. Irrigation lumen 14 is also formed in parallel to suction lumen 13 along the axial direction of main body tube portion 10. Furthermore, irrigation side hole 12 is connected to this irrigation lumen 14.

Among the 3 branch tubes branching from the base of the main body tube portion 10, first branch 10a is connected to suction lumen 13 of main body tube portion 10, second branch 10b is connected to two balloon lumens 15 and third branch 10c is connected to the irrigation lumen 14. The end of first branch 10a is connected to a suction bag (not shown in the figure) through a connector 10d. Furthermore, as shown in Figure 1, a valve 10e is connected to the end of second branch 10b. Moreover, a check valve 10f is connected to the end of third branch 10c. This check valve 10f allows fluid to flow into third branch 10c from the outside and inhibits it from flowing to the outside from third branch 10c.

As shown in Figure 2, main body tube portion 10 has first maneuvering wire lumen 16a, second maneuvering wire lumen 16b and third maneuvering wire lumen 16c (collectively referred to as maneuvering wire lumens 16, below) formed with equal spacing around the periphery of suction lumen 13 along the axis. These maneuvering wire lumens 16a, 16b and 16c respectively have flexible first maneuvering wire 17a, second maneuvering wire 17b and third maneuvering wire 17c (collectively referred to as maneuvering wires 17, below).

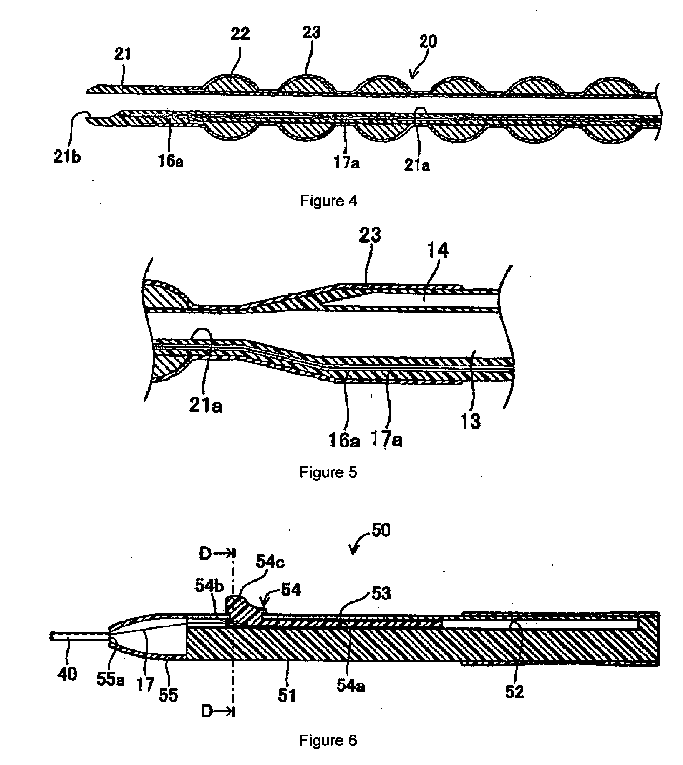

Figure 3 is a cross section along the axis of ileus tube 100 shown in Figure 1, including the central axis of main body tube portion 10 and guide portion 20. Figure 4 is an enlarged cross section of the vicinity of the guide portion 20 shown in Figure 3 (portion B in the figure), and Figure 5 is an enlarged cross section of the vicinity of the joint between the main body tube portion 10 and guide portion 20 shown in Figure 3 (portion C in the figure). As shown in Figure 3 and Figure 4, guide portion 20 is equipped with a guide tube 21 and weights 22. Guide tube 21 is a long tube with internal path 21 a. Path 21 a is connected to the outside through a tip opening 21 b formed at one end of guide tube 21 and, at the same time, to suction lumen 13 inside main body tube portion 10 on the other end of guide tube 21. Furthermore, the periphery of guide tube 21 has multiple (6 in this example) weights 22 installed with a constant interval along the axis. The weights 22 are in the form of spherical rings with perforated columnar hole, and each weight 22 is installed in a manner covering the periphery of guide tube 21. The periphery of these weights 22 is covered with a cover 23 made of a soft resin. This cover 23 restricts the axial movement of each weight 22, and weights 22 are fixed at required positions on guide tube 21.

Moreover, as apparent from Figure 2, Figure 3 and Figure 4, the first maneuvering wire lumen 16a is formed at a radial position deviating from the central axis of guide tube 21 and main body tube portion 10 along the axial direction from guide tube 21 to main body tube portion 10. One end (left end in Figure 4) of first maneuvering wire lumen 16a is closed near the tip of guide tube 21, and the other end is connected to the connection tube 40. One end (left end in Figure 4) of first maneuvering wire 17a protrudes from the closed end of first maneuvering wire lumen 16a and is attached inside the tube wall of guide tube 21. Incidentally, the other maneuvering wire lumens and maneuvering wires have the same configurations as those of first maneuvering wire lumen 16a and first maneuvering wire 17a; thus explanation is omitted.

As shown in Figure 3 and Figure 5, guide tube 21 is formed approximately coaxially with main body tube portion 10. Furthermore, the end of guide tube 21 close to main body tube portion 10 has a tapered shape, the outer diameter gradually increasing as approaching main body tube portion 10, and at the border with main body tube portion 10, it is connected without interruption to main body tube portion 10. Therefore, the outer diameter of guide tube 21 including the above tapered portion is smaller than that of main body tube portion 10. Furthermore, the thickness of the tube wall of guide tube 21 is thinner than that of main body tube portion 10. Furthermore, the material forming guide tube 21 is the same material used to form main body tube portion 10, and the main body tube portion 10 and guide tube 21 are formed as a unit. The above material is preferably a soft thermoplastic resin such as polyurethane, polyvinyl chloride, etc.

As shown in Figure 1, main body tube portion 10 has a connection tube 40. This connection tube 40 is connected to the end of first maneuvering wire lumen 16a, second maneuvering lumen 16b and third maneuvering wire 16c, and the other end is connected to a tensioning means 50. Maneuvering wires 17a, 17b and 17c inside maneuvering wire lumens 16 connect to tensioning means 50 through connection tube 40.

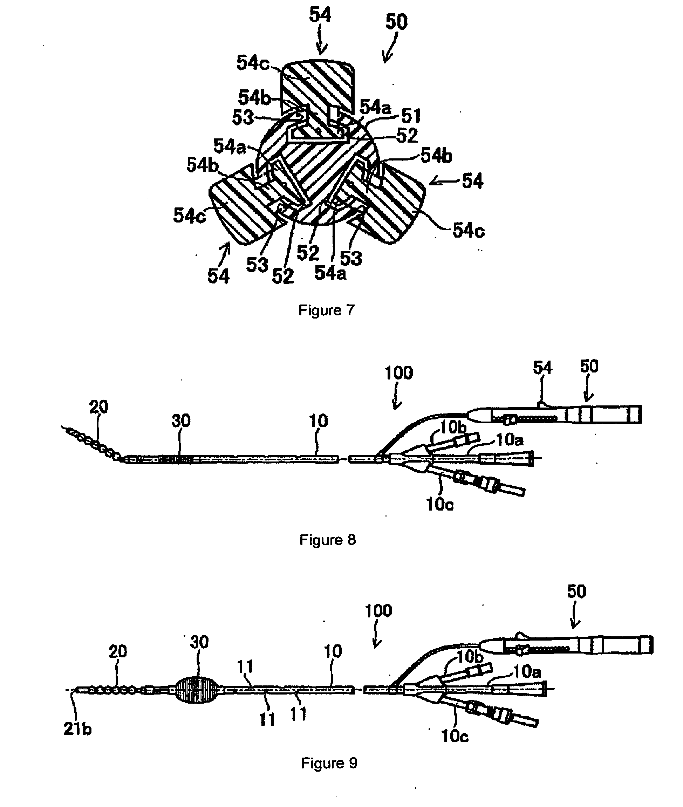

Tensioning means 50 has an elongated structure like a ballpoint pen in the example shown in Figure 1. Figure 6 is an axial cross section of tensioning means 50 including the longitudinal axis, and Figure 7 is a cross section of Figure 6 along line D-D. As is apparent from these figures, the tensioning means 50 has a rod-shaped main body 51. This main body 51 has three paths 52 formed along the axial direction with equal spacing in the peripheral direction. Furthermore, the periphery of main body 51 has a slit window 53 opening along the axial direction. This window 53 is opened at 3 sites with equal spacing facing the paths 52 so that they connect to paths 52, and prescribed portions (left half of path 52 in Figure 6) of paths 52 open to the outside. Furthermore, the width of windows 53 is set narrower than that of paths 52.

Main body 51 has a maneuvering lever 54. This maneuvering lever 54 has a guide 54a, connection 54b and operation part 54c. Guide 54a is inserted in path 52 and is movable along the axial direction of main body 51 via path 52. Connector 54b protrudes from guide 54a and extends outward from window 53 in the radial direction of main body 51. Operation part 54c is connected to the protruding portion of connector 54b. Therefore, operation part 54c may be operated with a finger to move along window 53, moving guide 54a in path 52. Incidentally, 3 maneuvering levers 54 are installed for the 3 paths 52, with equal spacing in the radial direction of main body 51.

As shown in Figure 6, the tip of main body 51 has a cap 55. Cap 55 is formed as a hollow bullet-shaped structure, the large diameter side connected to the end of main body 51, and opening 55a formed on the other side of a small diameter. This opening 55a is connected to one end of connection tube 40. The connection tube 40 has 3 maneuvering wires 17a, 17b and 17c, and these 3 maneuvering wires 17a, 17b and 17c enter paths 52 of main body 51 through the space inside cap 55 from connection tube 40. Furthermore, their ends are attached to guide 54a of the 3 operation levers 54. Therefore, it is possible to provide tension by sliding any of operation parts 54c to the right in the figure and pulling maneuvering wire 17 attached to operation lever 54. Incidentally, it is possible to install a biasing means such as a spring, etc., in path 52, where maneuvering lever 54 may be biased with this biasing means, allowing operation part 54c to be positioned regularly on the left end in Figure 6.

lleus tube 100 having the configuration described in detail above is used to carry out treatment or diagnosis of intestinal blockage as follows. First of all, ileus tube 100 is inserted nasally or orally into the body, guide portion 20 first. As a result, ileus tube 100 passes through the esophagus to reach the stomach, and furthermore, attempts to pass the stomach through the pyloric ring. To pass through the pyloric ring in this case, the operator maneuvers tensioning means 50 by maneuvering operating lever 54 to shift operation part 54c to the right in Figure 6. As a result, maneuvering wire 17 connected to one end of the maneuvering lever 54 is pulled, applying tension to the pulled maneuvering wire 17, and this tension is transmitted to guide tube 21. In this case, with the 3 maneuvering wire lumens 16a, 16b and 16c formed on the periphery of the suction lumen 13 at positions deviating from the central axis of main body tube portion 10 and guide tube 21, the tension applied to the maneuvering wire 17 is transmitted to the guide tube 21 in a manner deviating from the central axis. Therefore, guide tube 21 contracts toward the deviation of the tensioned maneuvering wire.

Moreover, guide tube 21 has a smaller outer diameter and thinner wall than that of main body tube portion 10, thus, is less rigid, than main body tube portion 10, and it is easily bendable. Therefore, as shown in Figure 8, the tension described above allows guide portion 20 alone to bend toward the deviation of the tensioned maneuvering wire. Guide portion 20 bends in the desired direction by carrying out the procedures described above, and consequently, the tip of guide portion 20 is made to point toward the pyloric ring. Ileus tube 100 is allowed to advance in this state, enabling ileus tube 100 to pass through the pyloric ring. Subsequently, procedures similar to those described above are carried out to pass through curved paths, including Treitz's ligament, and allow ileus tube 100 to reach the small intestine. If the curved direction of a path changes in this case, the operator of the tensioning means 50 can make the ileus tube 100 move in the direction of curvature of the path by suitably operating the maneuvering lever 54. In this case, two maneuvering levers 54 may be operated simultaneously to adjust the bending direction of the guide tube 21. For example, two of maneuvering levers 54 can be operated equally to bend guide portion 20 in directions not possible by operating the respective maneuvering levers 54 separately.

When main body tube portion 10 reaches the small intestine, etc., a fluid is fed to balloon lumens 15 from second branch 10b. As a result, fluid is fed to balloon 30 through balloon lumens 15, balloon 30 expands as shown in Figure 9 and comes into contact with the inner wall of the small intestine, etc. As a result, the peristaltic motion of the small intestine is transmitted to balloon 30, and ileus tube 100 advances inside the small intestine as a result of this peristaltic motion. Eventually, ileus tube 100 reaches the desired site.

As soon as ileus tube 100 reaches the desired site, the suction pack connected to first branch 10a is operated, and the pressure inside suction lumen 13 becomes negative. As a result, the contents of the small intestine, etc., are taken into the suction lumen 13 from the aspiration holes 11 and tip opening 21 b of the guide tube 21. The contents of the small intestine, etc., taken in as described above are recovered in the suction bag through the suction lumen 13 and first branch 10a. Intestinal blockage, etc., may be treated by carrying out the procedures described above.

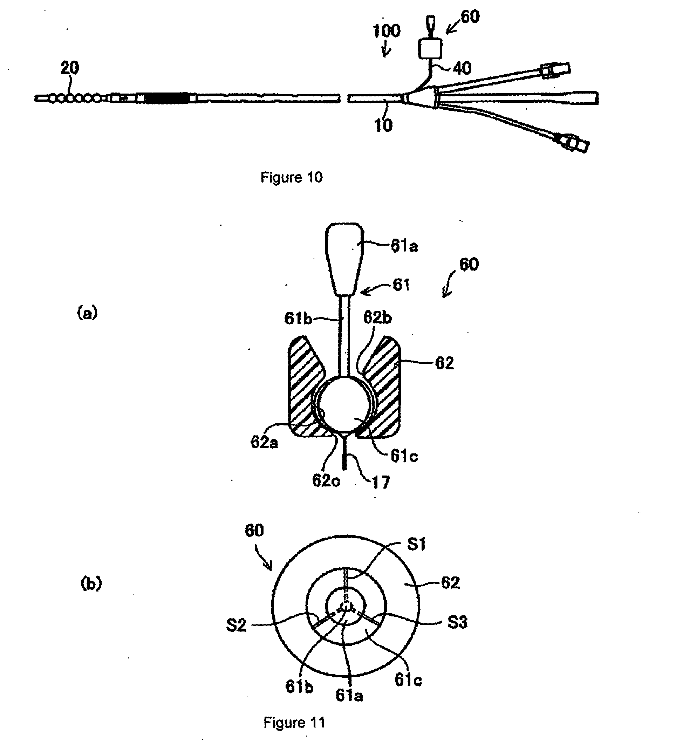

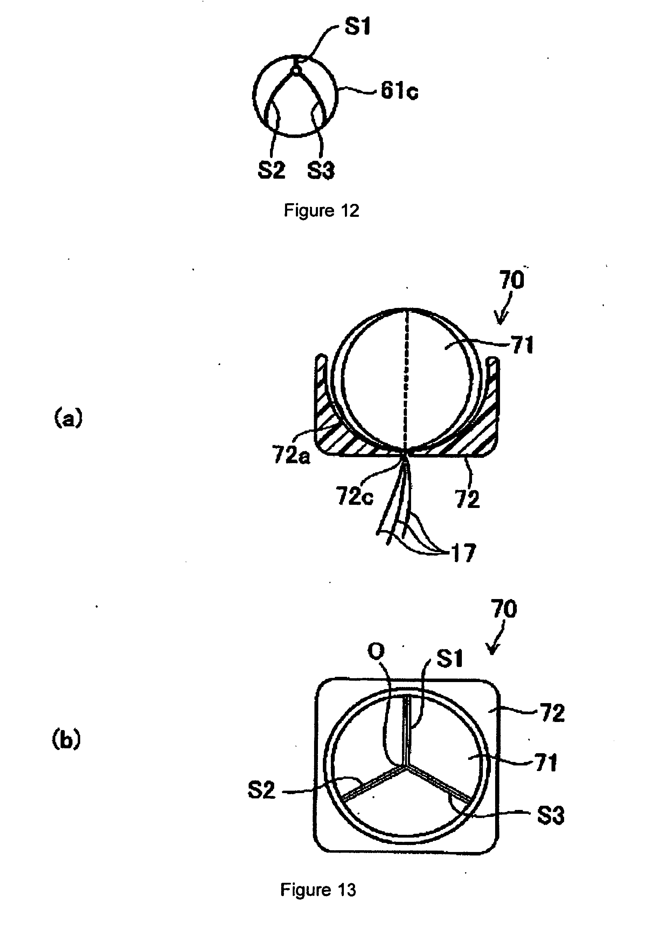

Figure 10 is a front view showing the whole ileus tube 100 having a tensioning means 60 different from tensioning means 50. Figure 11(a) is a cross section showing a portion of this tensioning means 60, and Figure 11(b) is a front view showing a portion. Tensioning means 60 is equipped with a maneuvering stick 61 and holder 62. Maneuvering stick 61 has a manual operation part 61 a, connection rod 61 b connected to operation part 61 a at one end and ball 61 c connected to the other side of connection rod 61 b. Ball 61 c is in the form of a sphere as shown in Figure 12, and 3 slits S1, S2 and S3 are formed from the connection with connection rod 61 b with equal spacing.

As shown in Figure 11(a), holder 62 has a spherical inner wall 62a forming a spherical space. The spherical space surrounded by this spherical inner wall 62a is connected to the outside at a top opening 62b and bottom opening 62c of holder 62. Ball 61 c is installed freely rotatably in this spherical space. In this case, connection rod 61 b, connected to ball 61 c, and operation part 61 a protrude from top opening 62b of holder 62.

On the other hand, from bottom opening 62c of holder 62, 3 maneuvering wires 17 passing through connection tube 40 are introduced to the spherical space inside holder 62. The 3 maneuvering wires 17 are positioned in slits S1, S2 and S3 formed on ball 61 c to restrict their positions on the surface of ball 61 c and, at the same time, attached to connection rod 61 b at the merging point of the 3 slits S1, S2 and S3. Therefore, the operation part 61 a is maneuvered to tilt the maneuvering stick 61, causing ball 61 c to rotate inside the spherical space, pulling one or two of the maneuvering wires among maneuvering wires 17 placed in slits S1, S2 and S3, tensioning them. It is possible to allow guide portion 20 to be bent by applying tension to one or two maneuvering wires by carrying out the above procedures. Tensioning means 60 of this example can control the tension applied to the 3 maneuvering wires 17 by maneuvering the single maneuvering stick 61. ln this case, if the tilting direction of maneuvering stick 61 and direction of guide part 20 to be bent are coordinated, it is possible to realize the direction of bending of guide portion 20 simply by feeling the tilting of maneuvering stick 61, achieving good maneuverability.

Furthermore, it is possible to configure the tensioning means as shown in Figure 13. In this example, the maneuvering stick of tensioning means 60 shown in Figure 11 is not installed; it is a tensioning means allowing the ball to be rotated directly with the palm of the hand. As shown in Figure 13, tensioning means 70 has a ball 71 and holder 72. Ball 71 has 3 slits S1, S2, S3 formed on the surface similarly to ball 61 c described above. Holder 72 has a semispherical inner wall 72a forming a semispherical space opened at the top. Furthermore, semispherical inner wall 72a has a bottom opening 72c formed at the lower portion of the figure. The lower half portion of ball 71 is placed freely rotatably in a semispherical space formed by semispherical inner wall 72a of holder 72, and the upper half is exposed from the top of holder 72. Maneuvering wires 17 are introduced into the semispherical space from bottom opening 72c and positioned in respective slits S1, S2 and S3 of ball 71. Maneuvering wires 17 are joined at the merging point O of slits S1, S2 and S3. In the tensioning means 70 of this configuration, ball 71 is allowed to rotate at the top with the palm of the hand, enabling control of the tension of the 3 maneuvering wires. Furthermore, if the rotating direction of ball 71 and bending direction of guide portion 20 are coordinated, it is possible to realize the direction of bending of guide portion 20 via the rotating of ball 71 simply by feel, achieving good maneuverability.

As explained above in detail, in ileus tube 100 in this embodiment of the present invention, the same material is used for the guide tube 21 and main body tube portion 10, and these two portions are formed as a unit. Moreover, the outer diameter of guide tube 21 is allowed to be smaller than the outer diameter of main body tube portion 10, making the guide tube 21 softer than main body tube portion 10, and consequently, only guide portion 20 is bendable when tensioning means 50, 60 or 70 is operated, tensioning the maneuvering wires 17. Consequently, it is possible to produce an ileus tube having a configuration allowing guide portion 20 to bend actively at a lowered manufacturing cost. In this case, if the outer diameter of main body tube portion 10 is A, and the outer diameter of guide tube 21 is B, the ratio A/B may be about ½. At this ratio, the difference in rigidity between main body tube portion 10 and guide tube 21 is significant, allowing only the guide portion 20 to be bent surely. Furthermore, if main body tube portion 10 and guide tube 21 are made of a material having a relatively low hardness such as polyurethane, the difference in rigidity between main body tube portion 10 and guide tube 21 becomes sufficiently large even if the ratio A/B is not so large, and consequently, it is possible to prevent any reduced durability of the guide tube 21 due to the outer diameter becoming too small.

Furthermore, the configuration has multiple weights 22 covering the outer periphery of guide tube 21. Consequently, guide portion 20 can be guided with weights 22 alone. In this case, ileus tube 100 is mainly guided with weights 22, the function of actively bending guide portion 20 is used as auxiliary, and consequently, the guiding of ileus tube 100 to the affected site effectively becomes easier than ever. Furthermore, as shown in Figure 3, outer diameter D1 at the base end of guide tube 21 is smaller than outer diameter D2 of the tip portion of guide tube 21. It is easier to bend at the base part of guide tube 21. Therefore, it is possible to bend guide portion 20 satisfactorily by slight maneuvering of the maneuvering wires. In addition, outer diameter D3 of the periphery of guide tube 21, where weights 22 are attached, is also smaller than outer diameter D2 of the tip of guide tube 21. Therefore, it is possible to reduce the outer diameter of weights 22, enabling reduced stress in the patients as a result of weights 22 of the ileus tube hitting the inner wall of a cavity in the body when the cavity is especially narrow, Moreover, there are multiple maneuvering wire lumens 16a, 16b and 16c formed from guide tube 21 to the main body tube portion 10, and respective maneuvering wires 17a, 17b and 17c are inserted in multiple maneuvering wire lumens 16a, 16b and 16c. Therefore, the guide portion may be bent in various directions by tensioning multiple maneuvering wires 17a, 17b and 17c.

The tensioning means 50 has a configuration in which the ends of multiple maneuvering wires 17a, 17b and 17c are connected individually to 3 maneuvering levers 54, and consequently, it is possible to bend the guide tube 21 in the desired direction by individually operating the 3 maneuvering levers 54 and tensioning the corresponding maneuvering wires. In tensioning means 60 or 70, the ends of all maneuvering wires 17a, 17b and 17c are connected to a single maneuvering means , that is, maneuvering stick 61 or ball 71, and the bending direction of the guide tube 21 may be determined by controlling the state of operation of the maneuvering means (tilt angle of the maneuvering stick 61 or state of rotation of the ball 71) and tensioning the corresponding maneuvering wires. Therefore, the control of tension applied to all of maneuvering wires 17a, 17b and 17c is manageable by a single maneuvering means enabling easy bending of guide tube 21.

## Claims

1. A medical tube (100) for insertion inside the body; comprising a main body tube portion (10) with a main interior lumen and guide tube portion, said guide tube portion having an outer tube diameter smaller than that of said main body tube and being of the same material as that forming said main body tube portion (10), said main body tube portion (10) and said guide tube portion being formed as a single body with said main body tube portion (10); the medical tube (100) further comprising maneuvering wire lumens (16) at radial positions displaced from a central axis of said guide and main tube portions said lumens extending from the guide tube portion to said main body tube portion (10) along an axial direction; and maneuvering wires inserted in the maneuvering wire lumens (16), wherein ends of said maneuvering wires are attached to said guide tube portion, **characterized in that** said guide tube portion comprises at least one weight (22) arranged around said guide tube portion.

2. The medical tube (100) of Claim 1, further comprising a tensioning device (50, 60, 70) arranged to apply tension to said maneuvering wires, said tensioning device (50, 60, 70) being connected to said maneuvering wires inserted in said maneuvering wire lumens (16).

3. The medical tube (100) of Claim 1, further comprising multiple tensioning devices (50, 60, 70) arranged to apply tension to said maneuvering wires inserted in said multiple maneuvering wire lumens (16).

4. The medical tube (100) according to any one of claims 1 to 3, wherein the medical tube (100) is an ileus tube (100) comprising a steerable distal portion and a main body portion, said main body portion comprising a plurality of lumens extending along a length of said main body portion including a central lumen extending from a distal end of the distal portion to a proximal end of the main body portion and maneuvering wire lumens (16) through which respective maneuvering wires pass, said maneuvering wires having a first end attached to said steerable distal portion and a second end attached to a tensioning device (50, 60, 70) at said proximal end of said main body portion, wherein said steerable distal portion and said main body portion are formed at least partly of the same material.

5. The medical tube (100) according to claim 4, wherein said steerable distal portion comprises at least one weight (22) positioned around said central lumen.

6. The medical tube (100) according to claim 5 wherein said steerable distal portion comprises a plurality of weights (22) positioned around said central lumen.

7. The medical tube (100) according to claim 6 wherein the plurality of weights (22) are equispaced apart.

8. The medical tube (100) according to claim 4 further comprising at least one inflatable balloon (30) positioned towards a distal end of said main body portion and wherein said at least one inflatable balloon (30) is connected to a lumen passing through said main body portion.

9. The medical tube (100) according to claim 4, wherein said main body portion includes an irrigation lumen (14) terminating at a distal end of said main body portion.

10. The medical tube (100) according to claim 9, wherein said main body portion includes a suction lumen (13) connected to aspiration holes (11) distributed along a length of said main body portion.

11. The medical tube (100) according to claim 4, wherein a single tensioning device (50, 60, 70) is attached to each maneuvering wire.

12. The medical tube (100) according to claim 11 wherein said single tensioning device (50, 60, 70) incorporates a movable joystick.

13. The medical tube (100) according to claim 4, wherein said maneuvering wires are attached to respective tensioning devices (50, 60, 70).

14. The medical tube (100) according to claim 13 wherein said tensioning devices (50, 60, 70) are accommodated in a housing.

15. The medical tube (100) according to claim 4 wherein the steerable distal portion has an outer diameter which is less than an outer diameter of said main body portion.

## Patentansprüche

1. Medizinscher Schlauch (100) zum Einführen in den Körper mit einem Hauptkörperschlauchteil (10) mit einem inneren Hauptlumen und einem Führungsschlauchteil, wobei der Führungsschlauchteil einen äußeren Schlauchdurchmesser aufweist, der kleiner ist als der des Hauptkörperschlauchs, und aus dem gleichen Material besteht wie der, aus dem der Hauptkörperschlauchteil (10) besteht, wobei der Hauptkörperschlauchteil (10) und der Führungsschlauchteil einstückig mit dem Hauptkörperschlauchteil (10) ausgebildet sind; wobei der medizinische Schlauch (100) weiterhin Manövrierdrahtlumen (16) in von einer mittleren Achse des Führungs- und des Hauptkörperschlauchteils versetzten radialen Positionen umfasst, wobei sich die Lumen in Axialrichtung von dem Führungsschlauchteil zum Hauptkörperschlauchteil (10) erstrecken, wobei Manövrierdrähte in die Manövrierdrahtlumen (16) eingesetzt sind, wobei Enden der Manövrierdrähte an dem Führungsschlauchteil befestigt sind, **dadurch gekennzeichnet, dass** der Führungsschlauchteil mindestens ein Gewicht (22) umfasst, das um den Führungsschlauchteil herum angeordnet ist.

2. Medizinischer Schlauch (100) nach Anspruch 1, weiterhin mit einer Spannvorrichtung (50, 60, 70), die zum Anlegen von Spannung an die Manövrierdrähte angeordnet ist, wobei die Spannvorrichtung (50, 60, 70) mit den in den Manövrierdrahtlumen (16) eingesetzten Manövrierdrähten verbunden ist.

3. Medizinischer Schlauch (100) nach Anspruch 1, weiterhin mit mehreren Spannvorrichtungen (50, 60, 70), die zum Anlegen von Spannung an die in den mehreren Manövrierdrahtlumen (16) eingesetzten Manövrierdrähte angeordnet sind.

4. Medizinischer Schlauch (100) nach einem der Ansprüche 1 bis 3, wobei der medizinische Schlauch (100) ein Ileus-Schlauch (100) ist, der einen lenkbaren distalen Teil und einen Hauptkörperteil umfasst, wobei der Hauptkörperteil mehrere Lumen umfasst, die sich entlang einer Länge des Hauptkörperteils erstrecken, einschließlich eines mittleren Lumens, das sich von einem distalen Ende des distalen Teils zu einem proximalen Ende des Hauptkörperteils erstreckt, und Manövrierdrahtlumen (16), durch die jeweiligen Manövrierdrähte passieren, wobei die Manövrierdrähte ein an dem lenkbaren distalen Teil befestigtes erstes Ende und ein an einer Spannvorrichtung (50, 60, 70) an dem proximalen Ende des Hauptkörperteils befestigtes zweites Ende aufweisen, wobei der lenkbare distale Teil und der Hauptkörperteil zumindest teilweise aus dem gleichen Material hergestellt sind.

5. Medizinischer Schlauch (100) nach Anspruch 4, wobei der lenkbare distale Teil mindestens ein Gewicht (22) umfasst, das um das mittlere Lumen herum angeordnet ist.

6. Medizinischer Schlauch (100) nach Anspruch 5, wobei der lenkbare distale Teil mehrere Gewichte (22) umfasst, die um das mittlere Lumen herum angeordnet sind.

7. Medizinischer Schlauch (100) nach Anspruch 6, wobei die mehreren Gewichte (22) äquidistant sind.

8. Medizinischer Schlauch (100) nach Anspruch 4, weiterhin mit mindestens einem aufblasbaren Ballon (30), der zu einem distalen Ende des Hauptkörperteils angeordnet ist, und wobei der mindestens eine aufblasbare Ballon (30) mit einem sich durch den Hauptkörperteil erstreckenden Lumen verbunden ist.

9. Medizinischer Schlauch (100) nach Anspruch 4, wobei der Hauptkörperteil ein Spüllumen (14) enthält, das an einem distalen Ende des Hauptkörperteils endet.

10. Medizinischer Schlauch (100) nach Anspruch 9, wobei der Hauptkörperteil ein Sauglumen (13) enthält, das mit Aspirationslöchern (11) verbunden ist, die entlang einer Länge des Hauptkörperteils verteilt sind.

11. Medizinischer Schlauch (100) nach Anspruch 4, wobei eine einzelne Spannvorrichtung (50, 60, 70) an jedem Manövrierdraht befestigt ist.

12. Medizinischer Schlauch (100) nach Anspruch 11, wobei die einzelne Spannvorrichtung (50, 60, 70) einen beweglichen Joystick enthält.

13. Medizinischer Schlauch (100) nach Anspruch 4, wobei die Manövrierdrähte an jeweiligen Spannvorrichtungen (50, 60, 70) befestigt sind.

14. Medizinischer Schlauch (100) nach Anspruch 13, wobei die Spannvorrichtungen (50, 60, 70) in einem Gehäuse untergebracht sind.

15. Medizinischer Schlauch (100) nach Anspruch 4, wobei der lenkbare distale Teil einen Außendurchmesser aufweist, der kleiner ist als ein Außendurchmesser des Hauptkörperteils.

## Revendications

1. Tube médical (100) destiné à l'insertion dans le corps ; comprenant une portion de corps de tube principal (10) avec une lumière intérieure principale et une portion de tube de guidage, ladite portion de tube de guidage ayant un diamètre de tube extérieur plus petit que celui dudit corps de tube principal et étant en le même matériau que celui formant ladite portion de corps de tube principal (10), ladite portion de corps de tube principal (10) et ladite portion de tube de guidage étant formée d'une seule pièce avec ladite portion de corps de tube principal (10) ; le tube médical (100) comprenant en outre des lumières (16) pour fils de manoeuvre dans des positions radiales espacées d'un axe central desdites portions de tube de guidage et de corps de tube principal, lesdites lumières s'étendant depuis la portion de tube de guidage jusqu'à ladite portion de corps principal le long d'une direction axiale ; et des fils de manoeuvre insérés dans les lumières (16) pour fils de manoeuvre, des extrémités desdits fils de manoeuvre étant attachées à ladite portion de tube de guidage, **caractérisé en ce que** ladite portion de tube de guidage comprend au moins un poids (22) agencé autour de ladite portion de tube de guidage.

2. Tube médical (100) selon la revendication 1, comprenant en outre un dispositif de tensionnement (50, 60, 70) prévu pour exercer une tension sur lesdits fils de manoeuvre, ledit dispositif de tensionnement (50, 60, 70) étant connecté auxdits fils de manoeuvre insérés dans lesdites lumières (16) pour fils de manoeuvre.

3. Tube médical (100) selon la revendication 1, comprenant en outre plusieurs dispositifs de tensionnement (50, 60, 70) prévus pour exercer une tension sur lesdits fils de manoeuvre insérés dans lesdites lumières (16) pour fils de manoeuvre.

4. Tube médical (100) selon l'une quelconque des revendications 1 à 3, dans lequel le tube médical (100) est un tube (100) pour iléus comprenant une portion distale dirigeable et une portion de corps principal, ladite portion de corps principal comprenant une pluralité de lumières s'étendant le long d'une longueur de ladite portion de corps principal, y compris une lumière centrale s'étendant depuis une extrémité distale de la portion distale jusqu'à une extrémité proximale de la portion de corps principal et des lumières (16) pour fils de manoeuvre à travers lesquelles passent des fils de manoeuvre respectifs, lesdits fils de manoeuvre ayant une première extrémité attachée à ladite portion distale dirigeable et une deuxième extrémité attachée à un dispositif de tensionnement (50, 60, 70) au niveau de ladite extrémité proximale de ladite portion de corps principal, ladite portion distale dirigeable et ladite portion de corps principal étant formées au moins en partie en le même matériau.

5. Tube médical (100) selon la revendication 4, dans lequel ladite portion distale dirigeable comprend au moins un poids (22) positionné autour de ladite lumière centrale.

6. Tube médical (100) selon la revendication 5, dans lequel ladite portion distale dirigeable comprend une pluralité de poids (22) positionnés autour de ladite lumière centrale.

7. Tube médical (100) selon la revendication 6, dans lequel lesdits poids (22) sont équidistants.

8. Tube médical (100) selon la revendication 4, comprenant en outre au moins un ballonnet gonflable (30) positionné vers une extrémité distale de ladite portion de corps principal et dans lequel ledit au moins un ballonnet gonflable (30) est connecté à une lumière traversant ladite portion de corps principal.

9. Tube médical (100) selon la revendication 4, dans lequel ladite portion de corps principal comporte une lumière d'irrigation (14) se terminant à une extrémité distale de ladite portion de corps principal.

10. Tube médical (100) selon la revendication 9, dans lequel ladite portion de corps principal comporte une lumière de succion (13) connectée à des trous d'aspiration (11) répartis le long d'une longueur de ladite portion de corps principal.

11. Tube médical (100) selon la revendication 4, dans lequel un dispositif de tensionnement (50, 60, 70) unique est attaché à chaque fil de manoeuvre.

12. Tube médical (100) selon la revendication 11, dans lequel ledit dispositif de tensionnement (50, 60, 70) unique comporte une manette de commande mobile.

13. Tube médical (100) selon la revendication 4, dans lequel lesdits fils de manoeuvre sont attachés à des dispositifs de tensionnement respectifs (50, 60, 70).

14. Tube médical (100) selon la revendication 13, dans lequel lesdits dispositifs de tensionnement (50, 60, 70) sont reçus dans un logement.

15. Tube médical (100) selon la revendication 4, dans lequel la portion distale dirigeable a un diamètre extérieur qui est plus petit qu'un diamètre extérieur de ladite portion de corps principal.
